# EUROPÄISCHE PATENTSCHRIFT

(11) **EP 3 762 165 B1**
(45) Veröffentlichungstag und Bekanntmachung des Hinweises auf die Patenterteilung: **12.07.2023**
(21) Anmeldenummer: 19719415.2
(22) Anmeldetag: 08.03.2019
(51) Int. Cl.: A61M 60/122, B22F 3/105, C04B 35/56, C04B 35/645, C22C 29/06, C22C 29/08

(54) **VERFAHREN ZUR HERSTELLUNG VON VERBUNDWERKSTOFFEN AUF BASIS VON WOLFRAMCARBID MIT EDELMETALLBINDERN**
PROCESS FOR PRODUCTION OF COMPOSITE MATERIALS BASED ON TUNGSTEN CARBIDE WITH PRECIOUS METAL BINDERS
PROCÉDÉ DE FABRICATION DE MATÉRIAUX COMPOSITES À BASE DE CARBURE DE TUNGSTÈNE AVEC LIANTS EN MÉTAUX PRÉCIEUX

(30) Priorität: 09.03.2018 DE 102018105489
(43) Veröffentlichungstag der Anmeldung: 13.01.2021
(73) Patentinhaber: Universität Rostock, 18051 Rostock (DE); HNP Mikrosysteme GmbH, 19053 Schwerin (DE)
(72) Erfinder: DAMERAU, Carsten, 19079 Banzkow (DE); BODNAR, Wiktor, 17121 Loitz (DE); BURKEL, Eberhard, 18055 Rostock (DE)
(74) Vertreter: Müller Schupfner & Partner Patent- und Rechtsanwaltspartnerschaft mbB
(86) Internationale Anmeldenummer: PCT/DE2019/100210
(87) Internationale Veröffentlichungsnummer: WO 2019/170200

(56) Entgegenhaltungen:
- EP-A1- 3 147 377
- EP-A1- 3 165 782
- WO-A1-2018/003877
- US-A- 3 049 753
- US-A1- 2006 141 093
- US-A1- 2014 191 443
- US-A1- 2015 176 106

## Beschreibung

### Einleitung

Die Erfindung betrifft ein Verfahren zur Herstellung eines Verbundwerkstoffes auf Wolframcarbid-Basis gemäß des Patentanspruchs 1. Es wurde gefunden, dass mit dem erfindungsgemäßen Verfahren besonders harte und gleichzeitig biokompatible Carbidhartmetalle auf Basis von Wolframcarbid unter Verwendung von Gold, Platin und/oder Palladium durch Sintern hergestellt werden können. Unter anderem können diese zur Verwendung als nickelfreier, verschleißbeständiger Konstruktionshartstoff für lebensmitteltechnische oder pharmazeutische Maschinenkomponenten, z.B. Prallplatten, Düsen, Blenden sowie als medizintechnischer Werkstoff für Implantate oder Bearbeitungswerkzeuge z.B. Messer, Scheren genutzt werden. Weiterhin können diese Carbidhartmetalle als Beschichtung von Werkzeugen und Prothesen sowie als Vollkörper für Pumpen, z.B. in der Medizintechnik eingesetzt werden.

### Stand der Technik

Kennzeichnend für Carbidhartmetalle sind eine sehr hohe Härte, insbesondere bei erhöhter Temperatur, und eine gute Verschleißfestigkeit. Wolframcarbide zeichnen sich besonders durch eine extreme Härte sowie durch eine sehr gute elektrische Leitfähigkeit und auch Wärmeleitfähigkeit aus. Aufgrund der hohen Verschleißresistenz sind solche Hartmetalle vielseitig einsetzbar und werden besonders als Werkstoff für Maschinen-, Stanz- und Schneidewerkzeuge in der Industrie genutzt.

Da Wolframcarbid beim Schmelzen zerfällt, können Wolframcarbid enthaltende Formkörper nur über Sinterprozesse hergestellt werden, wobei häufig ein kombiniertes Sinter/HIP Verfahren eingesetzt wird. Zur Beeinflussung der Härte, der Plastizität und der Bruchzähigkeit werden solche Hartmetallcarbide mit unterschiedlichen Metallbinderzusätzen wie Eisen, Cobalt und Nickel wegen der guten Benetzbarkeit hergestellt.

Ein typisches Wolframcarbid-Hartmetall besteht häufig aus 85 bis 94 % Wolframcarbid als Verstärkungsphase (Hauptphase) und 6 bis 15 % Cobalt oder Nickel (Bindemittelphase). Das Bindemittel füllt die Kornzwischenräume. Statt oder in Ergänzung zu Cobalt und Nickel sind aber auch Chrom als Bestandteil der Bindemittelphase vorgeschlagen worden. Die EP 0028620 B1 nennt z.B. neben Nickel und Chrom Ti, Zr, Hf, V, Nb und Ta als mögliche Bestandteile der Bindemittelphase.

Wolframcarbid-Hartmetall mit Co/Ni in der Binderphase neigt zur Entbindung und Versprödung in Kontakt mit Säuren bei einem pH < 4 oder wenn diese Legierungen in Kontakt mit Oxidationsmitteln oder Komplexbildnern kommen. Hierbei tritt eine Kontamination der Umgebung mit Ni und/oder Co auf, weshalb das potentielle Einsatzgebiet für WC-Co/Ni-Hartmetalle eingeschränkt ist.

Diese Eigenschaft schränkt z.B. den Einsatz derartiger Hartmetallteile an Sägen zur Frischholzbearbeitung oder Fräswerkzeugen im Bergbau erheblich ein. Im Weiteren schließt diese Eigenschaft und die geringe Biokompatibilität von Co und Ni den Einsatz in medizinischen, biologischen, lebensmittelnahen und pharmazeutischen Anwendungen praktisch aus. Aufgrund des Vorhandenseins von Co und/oder Ni in der Bindemittelphase und der damit verbundenen Zytotoxizität finden Hartmetalle auf WC-Basis bisher keine Anwendung als Werkstoff für medizinische Produkte oder Implantate.

Ein Implantat ist ein im Körper eingepflanztes künstliches Material, das permanent oder zumindest für einen längeren Zeitraum dort verbleiben soll. Der Bedarf an Implantaten und die Anforderungen an die Funktionalität und Biokompatibilität sind in den letzten Jahren ständig gestiegen. Von Implantaten wird gefordert, dass diese die Fähigkeit aufweisen, schnell in den Knochen einzuwachsen und eine gute Verbindung mit dem Knochen einzugehen. Moderne Implantate sollen mechanisch stabil sein und sich optimal und in kurzer Zeit mit dem körpereigenen Gewebe verbinden, wobei keine Abstoßungsreaktion oder gar Infektion hervorgerufen werden sollen. Nickel und Nickelionen jedoch sind als Kontaktallergen einzustufen, weshalb nickelhaltige Metallcarbide nicht als Implantate eingesetzt werden können.

Selbiges trifft auch auf medizinische Werkzeuge oder Maschinen zu. So dürfen etwa extrakorporale Blutpumpen, wie sie in der Medizintechnik benötigt werden, aufgrund der Zytotoxizität kein Cobalt oder Nickel enthalten.

Die meisten Anwendungen in der Industrie stehen im Zusammenhang mit der Härte und Verschleißfestigkeit der Hartmetallcarbide. Durch diese Eigenschaft sind z.B Wolframcarbide ein geeignetes Material zur Beschichtung von Arbeitsflächen von Werkzeugen. Allgemein bekannte Anwendungen in der Industrie sind die Beschichtung der Arbeitsflächen hoch beanspruchter Werkzeuge mit Wolframcarbid-Hartmetallen.

Da Wolframcarbid beim Schmelzen zerfällt, können Wolframcarbid enthaltende Formkörper nur über Sinterprozesse hergestellt werden, wobei häufig ein kombiniertes Sinter/HIP Verfahren eingesetzt wird.

DE 3128997 A1 offenbart die Herstellung von WC und Edelmetall enthaltenden Verbundwerkstoffen, welche mittels des sog. HIP-Verfahrens hergestellt werden. Es werden jedoch keine Verbundwerkstoffe offenbart, welche mind. 80 Gew.% aus WC und mind. 2 Gew.% eines Edelmetalls enthalten. Darüber hinaus führt das dort beschriebene Verfahren zu Materialien für die dort offenbarten WC und Edelmetall-Anteile zu Verbundwerkstoffen, welche eine substantielle Menge an W₂C als Verunreinigung enthalten.

Bekannt ist in der Pulvermetallurgie das sogenannte feldgestützte Sintern (FAST), welches häufig auch als Spark Plasma Sintern (SPS) oder gepulstes Stromsintern (PECS) bezeichnet wird. Dies ist ein neuartiges druckunterstütztes Sinterverfahren das mit gepulstem Gleichstrom arbeitet. Die Pulverproben werden für sehr kurze Zeitperioden (Minuten anstatt Stunden oder Tagen) dieser Energiezufuhr ausgesetzt. Im Verlauf der FAST Behandlung kann Pulver, welches in einer Form enthalten ist, zu verschiedenen neuartigen Körpern verarbeitet werden, zum Beispiel zu nanostrukturierten Materialien, Feinkeramiken, porösen Materialien usw.

US 3049753 A, EP 3147377 A1, US 2006/141093 A1 offenbaren Werkstoffe auf Wolframcarbidbasis, welche mittels herkömmlichen Sintervefahren hergestellt sind und folglich einen Anteil von W2C von mehr als 1 Gew.% vorliegt.

US 2015/176106 A1 offenbart ein Verfahren zur Herstellung eines WC-Werkstoffes, welches mittels FAST-Verfahren herstellbar ist und Cobalt in der Binderphase aufweist.

### Aufgabe der Erfindung

Aufgabe der Erfindung ist ein Verfahren bereitzustellen, mit dem ein Verbundwerkstoff auf Wolframcarbid-Basis hergestellt werden kann, welcher hohe Härte, Elastizität, Korrosionsfestigkeit, Basen- und Säurebeständigkeit und Biokompatibilität mit einem geringen technischen Herstellungsaufwand verbindet.

### Zusammenfassung der Erfindung

Erfindungsgemäß wird die Aufgabe entsprechend den Merkmalen des unabhängigen Patentanspruchs gelöst. Bevorzugte Ausgestaltungen sind Gegenstand der Unteransprüche oder nachfolgend beschrieben. Gegenstand der Erfindung ist ein Verfahren zur Herstellung eines Verbundwerkstoffs auf Wolframcarbid-Basis, d.h. umfassend überwiegend Wolframcarbid und daneben weiterhin zumindest ein oder mehrere Edelmetalle ausgewählt aus der Gruppe Gold, Palladium und Platin, wobei der Verbundwerkstoff 80 Gew.% bis 98 Gew.% Wolframcarbid und 2 Gew.% bis 20 Gew.% Edelmetalle aufweist bzw. enthält. Die Verbundwerkstoffe sind Hartmetalle.

Gesinterte Verbundwerkstoffe auf Wolframcarbid-Basis mit einem oder mehreren der Edelmetalle Au, Pd oder Pt in der Binderphase = WC/(Au, Pd, Pt) zeigen sich überraschenderweise sowohl im stark sauren Milieu als auch gegenüber Komplexbildnern und Oxidationsmitteln resistent, und sind aufgrund des NichtVorhandenseins zytotoxischer Co- und/oder Ni-Ionen eine interessante Alternative zu den bisher gängigen Wolframcarbiden. Diese Eigenschaft eröffnet mit einer Gold- oder Platin- oder Palladium-Binderphase ausgestatteten Wolframcarbid neue Einsatzmöglichkeiten. Derlei Hartmetalle finden insbesondere Einsatz als Vollkörper in medizinischen Produkten wie Blutpumpen und Prothesen und in lebensmitteltechnischen oder pharmazeutischen Maschinenkomponenten, z.B. Prallplatten, Düsen, Blenden oder Legierung für Werkzeuge, wie z.B. Sägen, Drehwerkzeuge, Werkzeuge zum Abstechen und Einstechen, Fräs- Bohr- oder Reibwerkzeuge.

Der Einsatz der WC/(Au, Pd, Pt)-Verbundwerkstoffe in Umgebungen mit pH < ca. 4 führt anders als bei der Verwendung von Co und/oder Ni in der Bindemittelphase nicht zur Versprödung oder Kontamination der Umgebung mit Fremdmetallionen wie Co- und/oder Ni-Ionen. Der gleiche Effekt zeigt sich in Kontakt mit Flüssigkeiten, die Komplexbildner oder Oxidationsmittel enthalten.

Die WC/(Au, Pd, Pt)-Verbundwerkstoffe, ggf. auch als Beschichtung, finden Anwendung in der Ausstattung von Arbeitsflächen von Bearbeitungswerkzeugen, z.B. für das Zerspanen wie Drehen, Fräsen, Bohren, Sägen und Schleifen, oder die spanlose Formgebung, wie z.B. das Tiefziehen, Scheiden, Stanzen oder Walzen.

Insbesondere betrifft die vorliegende Erfindung ein Verfahren zur Herstellung von Pumpenköpfen für Blutpumpen, wie sie in der Medizintechnik benutzt werden, sowie permanente Implantate beschichtet mit WC/(Au, Pd, Pt)-Verbundwerkstoffen, die in der plastischen oder orthopädischen Chirurgie als Ersatz für angegriffene oder zerstörte Körperteile, insbesondere Knochen oder als Dentalimplantate verwendet werden.

Nach der vorliegenden Erfindung wird ein als feldgestütztes Sintern (FAST) bezeichnetes Verfahren für die Herstellung der WC/(Au, Pd, Pt)-Verbundwerkstoffe angewandt. Das ist ein druckunterstütztes Sinterverfahren mit gepulstem Gleichstrom. Die Ausgangsmaterialien für die Metallcarbid-Herstellung im FAST Verfahren sind zumindest reines oder solches technischer Qualität, mit beispielsweise Chromcarbid als Kornwachstumsinhibitor, Wolframcarbidpulver und reines Gold-, Palladium- und/oder Platinpulver, die in der Kugelmühle als Mischung aufbereitet werden. In diesem Prozess werden die Materialien gemischt und/oder zerkleinert zu Partikelgrößen im Bereich von mehreren Mikrometern bis hin zu einigen Nanometern. Es können aber auch nasschemisch oder mittels beispielsweise CVD beschichtete Metallcarbidpulver eingesetzt werden. Die aus den Materialien nach dem erfindungsgemäßen Verfahren hergestellten Verbundwerkstoffe enthalten vorzugsweise zu weniger als 3 Atom% Fremdatome (andere Atome als W, C, Au, Pd und/oder Pt), wie bspw. das o.g. Chromcarbid als Kornwachstumsinhibitor.

### Detaillierte Beschreibung der Erfindung

Die WC/(Au, Pd, Pt)-Verbundwerkstoffe sind über Sinterprozesse zugänglich.

Erfindungsgemäß wird das FAST-Verfahren für die Herstellung von WC/(Au, Pd, Pt)- Verbundwerkstoffen eingesetzt. Die Vorteile des FAST Verfahrens im Vergleich zu Verfahren mit hohem Druck oder hohen Temperaturen für die Wolframcarbid-Verdichtung sind ein vergleichbar niedriger Druck auf der MPa Skala und eine hohe Effektivität mit einer hohen Aufheizgeschwindigkeit bis hin zu etwa 1000 K/min mit Hilfe gepulster Gleichströme im Bereich mehrerer Tausend Ampere, einer Haltezeit von wenigen Minuten und einer kurzen Abkühlphase. Die hier vorgeschlagene Methode kann für die energieeffiziente Herstellung angewendet werden.

Ein großer Vorteil des FAST-Verfahrens für die Herstellung von WC/(Au, Pd, Pt)-Verbundwerkstoffen liegt weiterhin in der kurzen Prozesszeit begründet. Dies führt zu einer Reduzierung des Kornwachstums im Sinterprozess, wodurch eine Nanostruktur in der Körnung des Werkstoffes beibehalten wird. Dies hat positive Auswirkungen auf die mechanischen Eigenschaften des Materials.

Weiterhin entsteht bei der Herstellung der WC/(Au, Pd, Pt)- Verbundwerkstoffe mit der FAST Methode nur ein sehr geringer Anteil einer W₂C-Phase von kleiner 1 Gewichts %, die normalerweise in konventionellen Sinter/HIP Verfahren oftmals gebildet wird. Der mit der W₂C-Phase verbundene negative Einfluss auf die mechanischen Eigenschaften der Werkstoffe wird durch die hier vorgeschlagene Herstellungsmethode stark reduziert.

Bei der FAST-Methode wird das zu verarbeitende Material zunächst in eine Matrize eingebracht und dann gepresst. Zur Wärmeeinbringung fließt ein gepulster Gleichstrom direkt durch die Matrize und die Probe, dessen Stromstärke und Spannung abhängig von der elektrischen Leitfähigkeit der Komponenten, ihrer Größe und der momentanen Sintertemperatur ist. Für elektrisch leitende Materialien wird eine signifikante Steigerung der Verdichtungsrate durch den Einfluss des elektrischen Feldes und des Stromflusses erzielt. Der kompakte Aufbau des Presswerkzeuges ermöglicht es, Heizraten bis etwa 1000 K/min zu erreichen.

Die vorverdichteten Pulver werden in die FAST-Kammer eingebracht und dann z.B. unter einem uniaxialen Druck von 10 MPa bis 300 MPa, insbesondere 50 MPa bis 120 MPa, in einem Vakuum oder einer Schutzgasatmosphäre durch den gepulsten Gleichstrom auf 1000°C bis 2000°C, insbesondere 1400°C bis 1800°C erhitzt.

Im Laufe des FAST Verfahrens wird typischerweise eine Stromstärke von 0.5 kA bis 10 kA gewählt. Die Spannung ist dabei relativ niedrig, z.B. eine Spannung von unter 10 V.

Die Vorteile des FAST Verfahrens im Vergleich zu Verfahren mit hohem Druck oder hohen Temperaturen für die Kompaktierung sind ein niedriger Druck auf der MPa-Skala und eine hohe Effektivität mit einer hohen Aufheizgeschwindigkeit bis etwa 1000 K/min und vorzugsweise von größer 100 K/min, einer Haltezeit von wenigen Minuten, z.B. kleiner 20 min, und einer kurzen Abkühlphase, wobei aber auch ohne Haltezeit (0 min) direkt in die Abkühlphase übergegangen werden kann. Die hier vorgeschlagene Methode kann für die energieeffiziente Herstellung angewendet werden.

Dies führt zu einer Reduzierung des Kornwachstums im Sinterprozess, wodurch eine Nano- und Mikrostruktur in der Körnung des Werkstoffes beibehalten wird. Dies hat positive Auswirkungen auf die mechanischen Eigenschaften des Materials.

WC-Verbundwerkstoffen sind Hartmetalle mit Au-, Pd- und/oder Pt-Binderzusätzen und zeichnen sich durch ihre mechanischen Eigenschaften und insbesondere ihre hohe Härte aus. Bei der hier vorgeschlagenen Herstellungsmethode eines WC/(Au, Pd, Pt)- Verbundwerkstoffes ist beim Einsatz von nanometergroßen Pulverkörnern die Größenverteilung der Körner im Sinterendprodukt sehr gut kontrollierbar. Aufgrund der kurzen Prozesszeiten tritt kaum unkontrolliertes Kornwachstum ein. Durch die Erhaltung der Nanostrukturierung zeigt das Elastizitätsmodul für die mit der FAST-Methode gesinterten Werkstoffe zwar keine signifikanten Veränderungen gegenüber konventionell hergestellten WC/(Au, Pd, Pt)-Verbundwerkstoffen, aber eine deutlich höhere Härte.

Bei der hier vorgeschlagenen Herstellungsmethode eines WC/(Au, Pd, Pt)-Verbundwerkstoffes wird auf den Einsatz von Co und Ni als Binderphase verzichtet. Dies führt zu einer hohen Biokompatibilität des Hartmetalles, was negative Auswirkungen des Materials auf den menschlichen Organismus beim Einsatz im Medizinsektor, der Industrie oder im alltäglichen Haushalt reduziert. Insbesondere, wenn der Werkstoff im medizinischen Sektor angewandt werden soll, ist der Verzicht auf Bindemittel wie Cobalt oder Nickel von großem Vorteil, denn die geringe Biokompatibilität von Co und Ni schließt den Einsatz in medizinischen, biologischen, lebensmittelnahen und pharmazeutischen Anwendungen praktisch aus. Der Einsatz in Umgebungen mit pH < ca. 4 führt zu Entbinderung, Versprödung und Kontamination der Umgebung mit Co- und Ni-Ionen. Der gleiche Effekt stellt sich in Kontakt mit Flüssigkeiten ein, die Komplexbildner oder Oxidationsmittel enthalten. Die nach dem erfindungsgemäßen Verfahren hergestellten WC/(Au, Pd, Pt)- Verbundwerkstoffe zeigen daher eine höhere Biokompatibilität.

In vitro Studien an isolierten Leukozyten und Lymphozyten aus dem menschlichen Blut zeigten, dass Hartmetallpartikel aus Co als auch WC-Co dosisabhängig Schaden an Chromosomen und DNA hervorrufen, während reines WC keine dosisabhängigen Schäden aufweist [F. Van Goethem et al., Mutation Research 392 (1997) 31-43]. Zytotoxizitätsstudien an menschlichen embryonalen Nierenzellen, menschlichen Neuroepitheliom-Zellen, Mausmyoblasten und Hippocampus primären neuronalen Kulturen von WC-Hartmetallen mit Co und Ni und von reinem W, Co und Ni haben gezeigt, dass Zytotoxizität bereits ab Konzentrationen von 50 ppm auftreten, und dass signifikante Toxizität von Ni und Co bei fast allen getesteten Konzentrationen auftritt [R. Verma et.al., Toxicology and Applied Pharmacology 253 (2011) 178-187].

Die Beschichtungen von Oberflächen können durch beispielsweise Sputtern, PVD, CVD, Laserablation oder direkt durch FAST-Sintern hergestellt werden. Insbesondere gibt FAST die Möglichkeit Sputtertargets aus WC/(Au, Pd, Pt)-Verbundwerkstoffen herzustellen. Der hergestellte Vollkörper kann dann entsprechend direkt weiter als Sputtertarget verwendet werden.

### Ausführungsbeispiel

Nachfolgend wird ein erfindungsgemäßes Verfahren der Herstellung eines gesinterten Wolframcarbid-Gold Verbundwerkstoffes an einem Beispiel erläutert:

### 1. Herstellung eines gesinterten Wolframcarbid-Gold Verbundwerkstoffes.

23.75 g Wolframcarbid- und 1.25 g Gold-Pulver wurden in einer Kugelmühle für 2 h bei 200 Umdrehungen pro Minute mit einem Kugel-zu-Pulver-Verhältnis von 10:1 in Hexan vermahlen. Das Pulvergemisch wurde getrocknet und anschließend in die Graphitform mit 20 mm Durchmesser gegeben. Das Pulver wurde in der Graphitform in der FAST-Kammer unter Vakuum verarbeitet. Der Anfangsdruck betrug 10 MPa. In den folgenden 15 min wurde der Druck von 100 MPa stetig aufgebaut. Der maximale angewandte gepulste Gleichstrom für das FAST Verfahren erreichte 2 kA und die Spannung 5.3 V. Das Prüfstück wurde mit einer Heizrate von 150 K/min auf eine Temperatur von 1600°C erhitzt. Die Haltezeit des Sintervorgangs bei 1600°C betrug 5 min. Danach wurde der Strom abgeschaltet, aber der Druck auf das Probenstück beibehalten. Nachdem der Sintervorgang beendet war, wurde das Probenstück mit Hilfe eines Sandstrahlers von eventuellen Graphitrückständen befreit.

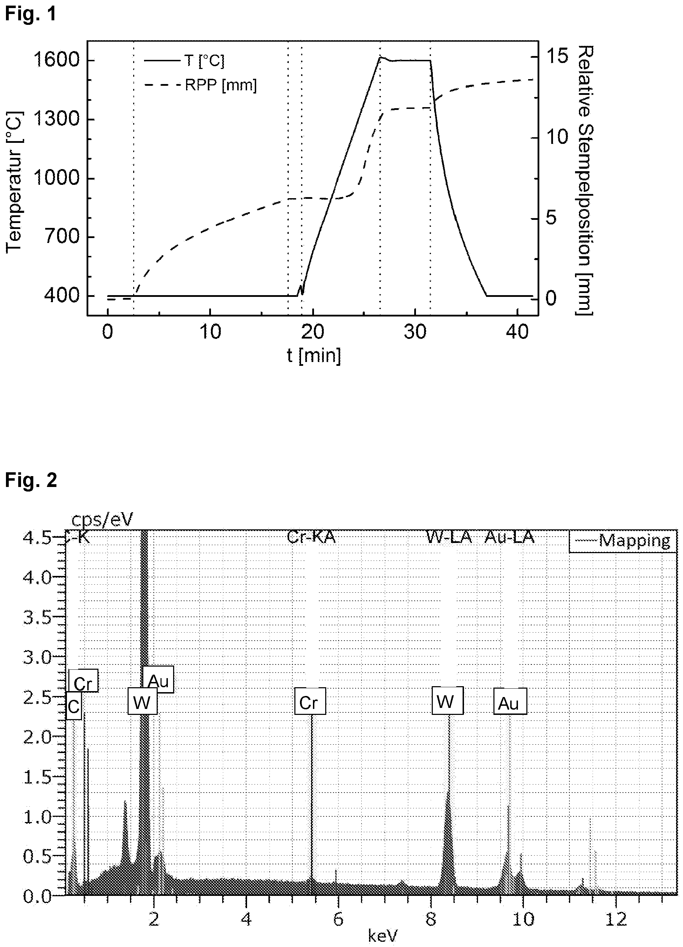

Der Verlauf des Sinterprozesses für den nach Beispiel 1 hergestellten Wolframcarbid-Gold Verbundwerkstoff mit 5 Gew.% Au ist in ***Fig.1*** dargestellt.

Durch den Sintervorgang wurde ein Wolframcarbid-Gold Verbundwerkstoffhergestellt, dessen relative Dichte bei 98.2% der theoretischen Dichte lag.

### 2. Struktur des Probenstücks nach Beispiel 1

Die Sinterung bei 1600°C ergab bei einer Ausgangsgröße des eingesetzten Pulvers von 100 nm eine mittlere Korngröße von 306 nm im gesinterten Werkstoff.

Die strukturellen Untersuchungen des Wolframcarbid-Gold Verbundwerkstoffes mit EDX (***Fig**. **2***) zeigen, dass nur W, C, Cr, und Au in der gesinterten Probe vorhanden sind, sodass es zu keiner Kontamination mit anderen Materialien durch den Herstellungs- und Bearbeitungsprozess kommt. ***Fig. 2*** zeigt insofern das Element-Mapping der inneren Bruchkante des Probenstücks nach Beispiel 1.

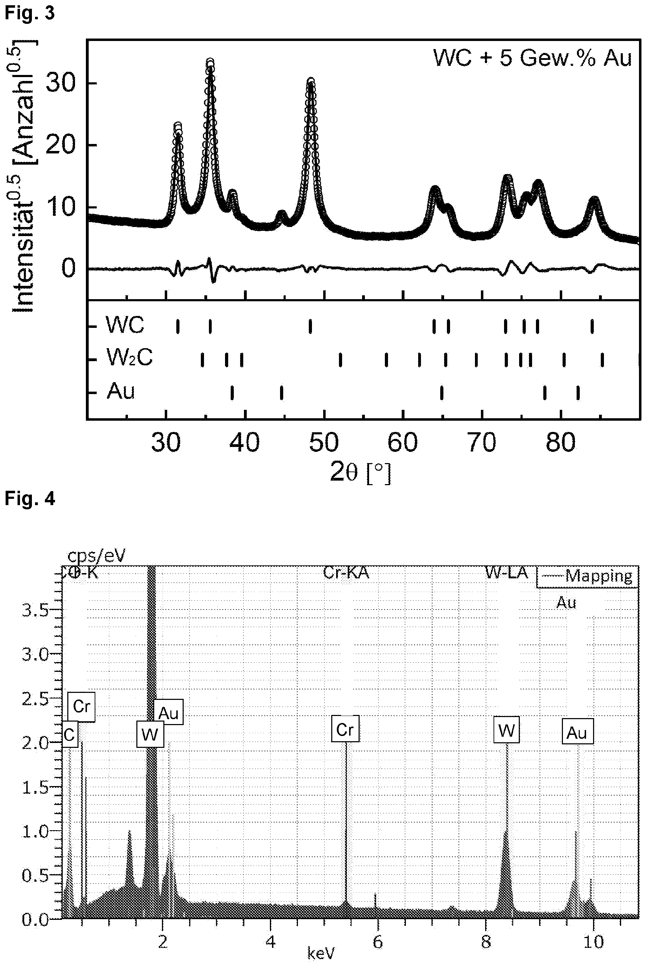

Das Röntgendiffraktogramm des Probenstücks (***Fig. 3***) bestätigt, dass durch das Herstellungsverfahren und das Einbinden von Gold eine Reduzierung der konventionell auftretenden W₂C-Phase erfolgt. Dies lässt sich an der Reduktion des Hauptpeaks von W₂C erkennen. Zusätzlich werden das berechnete Diffraktogramm und die Differenz zum gemessenen Diffraktogramm dargestellt. Die Bragg-Reflexe der auftretenden Phasen sind im unteren Teil der Abbildung 3 dargestellt.

### 3. Chemikalienbeständigkeit des Probenstücks nach Beispiel 1

Die Stabilität des Wolframcarbid-Gold Verbundwerkstoffes wurde durch die Behandlung mit Kaliumcyanid geprüft:

4 Au + 8 KCN + O₂ + 2 H₂O → 4 KAu(CN)₂+ 4 KOH

Das Waschen einer Probe in Kaliumcyanidlösung vermittelt einen Eindruck über die Beständigkeit des Werkstoffes. Hierzu wurde ein Teil des Probenstücks mit einer Masse m = 1,3705 g in 60 ml Wasser mit 100 mg KCN gelegt. Die Kaliumcyanidlösung mit dem Teil des Probenstücks wurde für 5 Tage ständig gerührt. Danach wurde die Probe wieder gewogen und es wurde eine Masse m* von 1,3665 g ermittelt.

Während des Experimentes war ein hoher Lufteintrag beobachtet worden. Da die Reduktion der Masse des Probestücks nicht signifikant war, wurde das Probenstück untersucht und EDX der Bruchkante durchgeführt. ***Fig. 4*** zeigt das Spektrum der aufgenommenen Röntgenstrahlung, das neben W, C und Cr immer noch einen merklichen Anteil an Au aufweist. Dies zeigt, dass scheinbar nur auf der Oberfläche der Probe ein kleiner Anteil des Golds abgelöst wurde. Innerhalb des Probenstücks hat sich die Struktur nicht geändert.

### 4. Biokompatibilität des Probenstücks nach Beispiel 1

Um die Biokompatibilität des Probenstücks nach Beispiel 1 zu bewerten, wurde das Prüfstück in eine simulierte Körperflüssigkeit mit einem pH-Wert von 7.25 für 8 Wochen bei 36.5°C eingelegt und permanent geschüttelt. Anschließend wurde die Lösung auf die vorhandenen Rückstände mit Hilfe der Atomemissionsspektroskopie untersucht.

Die Herstellung der simulierten Körperflüssigkeit und die Versuchsdurchführung ist in
- F. Zhang, E. Burkel. "Novel titanium manganese alloys and their macroporous foams for biomedical applications prepared by field assisted sintering", Biomedical Engineering, Trends, Researches and Technologies, Rejeka: InTech (2011) 203-224
näher beschrieben.

Eine Übersicht über die in der simulierten Körperflüssigkeitslösung nach Ende der Auslagerung gefundenen Rückstände von W und Au sind in der folgenden Tabelle dargestellt.

| Element | Wellenlänge [nm] | WC+5%Au [ppm] |
|---|---|---|
| W | 220.448 | 1.45 |
| W | 239.709 | 1.46 |
| Au | 242.795 | < 0.01 |
| Au | 267.595 | 0.03 |

Nach einem Langzeittest von 8 Wochen konnten nur Konzentrationen von weniger als 1.5 ppm in der Lösung nachgewiesen werden, was sich positiv auf die Anwendungen im medizinischen Sektor auswirken sollte.

### 5. Mechanische Eigenschaften des Probenstücks nach Beispiel 1

Die mechanischen Eigenschaften des Prüfstückes wurden in einem Nanoindenter mit der Berkovich-Methode und in einem Mikroindenter mit der Vickers-Methode untersucht.

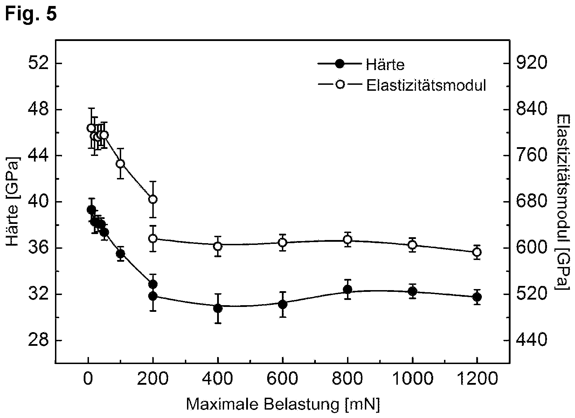

Das Elastizitätsmodul und die Härte der Prüfstücke sind ebenso in ***Fig. 5*** dargestellt. Die erhaltenen Daten, die durch Nanoindentierung (Belastungsbereich 50-200 mN) und Mikroindentierung (Belastungsbereich 200-1200 mN) ermittelt wurden, zeigen die hohe Härte und das ausgezeichnete Elastizitätsmodul des Probenstücks. Der Unterschied in den erhaltenen Nano- und Mikroindentierungsresultaten ist in der unterschiedlichen Methodik der Messungen begründet.

## Patentansprüche

1. Verfahren zur Herstellung eines Verbundwerkstoffs auf Wolframcarbid-Basis umfassend weiterhin zumindest ein oder mehrere Edelmetalle ausgewählt aus der Gruppe Gold, Palladium und Platin, wobei der Verbundwerkstoff 80 Gew.% bis 98 Gew.% Wolframcarbid und 2 Gew.% bis 20 Gew.% Edelmetalle und vorzugsweise weniger als 3 Atom% andere Atome enthält durch Sintern mittels der FAST-Methode, umfassend die folgenden Schritte
- Bereitstellen eines Pulvers bzw. einer Pulvermischung umfassend zumindest Wolframcarbid und eines oder mehrere der Edelmetalle,
- Aussetzen des Pulvers bzw. der Pulvermischung
- einer Spannung von unter 10 V
- einem Strom von 0.5 kA bis 10 kA und
- einem Druck von 10 MPa bis 300 MPa.

2. Verfahren nach Anspruch 1, wobei das Verfahren in einem Vakuum oder einer Schutzgasatmosphäre durchgeführt wird.

3. Verfahren nach Anspruch 1 oder 2, wobei das Pulver bzw. die Pulvermischung mit einer Aufheizrate bis 1000 K/min, vorzugsweise größer 100 K/min, auf insbesondere 1000°C bis 2000°C, vorzugsweise 1400°C bis 1800°C, bei einem Druck von 10 MPa bis 300 MPa, vorzugsweise 50 MPa bis 120 MPa erhitzt wird und danach abkühlt.

4. Verfahren nach einem der vorherigen Ansprüche zur Herstellung von Pumpenköpfen für Blutpumpen oder zur Herstellung permanenter Implantate beschichtet mit dem Verbundwerkstoff auf Wolframcarbid-Basis.

## Claims

1. A process for producing a composite material based on tungsten carbide furthermore comprising at least one or several noble metals selected from the group of gold, palladium and platinum, wherein the composite material contains 80 weight percent to 98 weight percent of tungsten carbide and 2 weight percent to 20 weight percent of noble metals and preferably less than 3 atomic percent of other atoms by means of sintering using the FAST method, comprising the following steps:
- providing a powder or a powder mixture, respectively, comprising at least tungsten carbide and one or several of the noble metals,
- exposing the powder or the powder mixture, respectively, to
- a voltage below 10 V
- a current of 0.5 kA to 10 kA, and
- a pressure of 10 MPa to 300 MPa.

2. The process according to claim 1, wherein the process is carried out in a vacuum or an inert gas atmosphere.

3. The process according to claim 1 or 2, wherein the powder or the powder mixture, respectively, is heated at a heating rate up to 1000 K/min, preferably greater than 100 K/min, to in particular 1000°C to 2000°C, preferably 1400°C to 1800°C, at a pressure of 10 MPa to 300 MPa, preferably 50 MPa to 120 MPa, and cools down afterwards.

4. The process according to any one of the preceding claims for producing pump heads for blood pumps or for producing permanent implants coated with the composite material based on tungsten carbide.

## Revendications

1. Procédé de fabrication d'un matériau composite à base de carbure de tungstène, comprenant en outre au moins un ou plusieurs métaux précieux choisis dans le groupe constitué par l'or, le palladium et le platine, le matériau composite contenant de 80 % à 98 % en poids de carbure de tungstène et de 2 % à 20 % en poids de métaux précieux et de préférence moins de 3 % d'autres atomes, par frittage au moyen du procédé FAST, comprenant les étapes suivantes :
- la mise à disposition d'une poudre et/ou d'un mélange de poudres comprenant au moins du carbure de tungstène et un ou plusieurs des métaux précieux,
- l'exposition de la poudre et/ou du mélange de poudres à
- une tension inférieure à 10 V
- un courant de 0,5 kA à 10 kA et
- une pression de 10 MPa à 300 MPa.

2. Procédé selon la revendication 1, le procédé étant réalisé sous vide ou dans une atmosphère de gaz inerte.

3. Procédé selon la revendication 1 ou 2, la poudre et/ou le mélange de poudres étant chauffé à une vitesse de chauffage allant jusqu'à 1000 K/min, de préférence supérieure à 100 K/min, en particulier à une température de 1000°C à 2000°C, de préférence de 1400°C à 1800°C, à une pression de 10 MPa à 300 MPa, de préférence de 50 MPa à 120 MPa, puis étant refroidi.

4. Procédé selon l'une des revendications précédentes pour la fabrication de têtes de pompe pour pompes à sang ou pour la fabrication d'implants permanents revêtus du matériau composite à base de carbure de tungstène.
